(19) Europäisches Patentamt
European Patent Office
Office européen des brevets

(11) Publication number: 0 243 869 B1

# (12) EUROPEAN PATENT SPECIFICATION

(45) Date of publication of patent specification: **17.06.92**

(51) Int. Cl.5: **C07C 37/62**, C07C 39/26

(21) Application number: **87105917.6**

(22) Date of filing: **22.04.87**

## (54) Process for the preparation of di-ortho-substituted di-meta-halogenated para-halomethylphenols.

(30) Priority: **30.04.86 US 858473**

(43) Date of publication of application:
**04.11.87 Bulletin 87/45**

(45) Publication of the grant of the patent:
**17.06.92 Bulletin 92/25**

(84) Designated Contracting States:
**AT BE CH DE ES FR GB IT LI NL SE**

(56) References cited:
**EP-A- 0 158 709**
**EP-A- 0 159 240**

**CHEMICAL ABSTRACTS, vol. 84, no. 7, 16th February 1976, page 443, column 1, abstract no. 43511u, Columbus, Ohio, US; N.V. LYUKSHOVA et al.: "Replacement of a methyl group in the bromination of some methylated phenols"**

**JOURNAL OF THE CHEMICAL SOCIETY, Section B, 1969, pages 373-377; G. ANTINORI et al.: "Non-conventional paths in electrophilic aromatic reactions. Part VI. Chlorination of 3, 5-dichloro-2,4,6-trimethylanisole and related compounds"**

**JOURNAL OF THE CHEMICAL SOCIETY, PERKIN TRANSACTIONS II, vol. 9, 1974, pages 1192-1195, London, GB; V. CALO et al.: "The ortho:para ratio in the bromination of phenol. Evidence for a coordination effect"**

**JOURNAL OF THE CHEMICAL SOCIETY, PERKIN TRANSACTIONS II, 1981, pages 32-41, London, GB; J.M. BRITTAIN et al.: "Electrophilic substitution with rearrangement. Part 9. Dienones derived from brominations of o-, m- and p-cresol"**

(73) Proprietor: **THE DOW CHEMICAL COMPANY**
**2030 Dow Center Abbott Road P.O. Box 1967**
**Midland Michigan 48640-1967(US)**

(72) Inventor: **Mendoza, Abel**
**3001 Gibson Street**
**Midland Michigan 48640(US)**

(74) Representative: **Sternagel, Hans-Günther, Dr. et al**
**Patentanwälte Dr. Michael Hann Dr. H.-G. Sternagel Sander Aue 30**
**W-5060 Bergisch Gladbach 2(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention concerns a process for the preparation of halogenated phenols. The halogenated phenols are useful flame retardants, fungicides and monomers.

"Halomethylation" of aromatic compounds is a well established reaction. However, halomethylation of phenols is generally not a selective process. In general, it is difficult to obtain monomeric chloromethylated products. See, G. A. Olah, Friedel-Crafts and Related Reactions, Vol. II, Part 2, pp. 701-11, 1964. Halogenation of alkylphenols is normally accompanied by side-reactions. See, V. V. Ershov, A. A. Volodkin & G. N. Bogdanov, Russion Chemical Reviews, 32, 75-93 (1963). Furthermore, bromomethylation, in general, affords lower yields than chloromethylation, ibid., pp. 734-735.

The preparation of a halogenated phenol such as 4-bromomethyl-3,5-dibromo-2,6-dimethylphenol (tribromomesitol) can involve the following sequence: bromination of 2,4,6-trimethylphenol (mesitol) with 2 moles of bromine in acetic acid to prepare 3,5-dibromo-2,4,6-trimethylphenol (dibromometisol) which is isolated by precipitation with water. K. Auwers & F. Rapp, Ann., 302, 153-71 (1898) and O. Jacobsen, Ann., 195, 265-92 (1879); the dibromomesitol is brominated in acetic acid sodium acetate buffer at low temperatures to afford an intermediate, 3,4,5-tribromo-2,4,6-trimethyl-2,5-cyclohexadienone, which is isolated by fast precipitation with water and filtration; this solid slowly rearranges to tribromomesitol at room temperature, more quickly at higher temperatures. K. Fries & E. Brandes, Ann., 542, 48-77 (1939). This procedure to prepare tribromomesitol is complex and not industrially viable.

A more direct method of preparation of tribromomesitol involves the reaction of mesitol in glacial acetic acid with an excess of bromine, using 10.6 moles of bromine per mole of mesitol. The yields are 66 percent and the product isolation is difficult. K. Auwers and H. Allendorff, Ann., 302, 76-98 (1898). This procedure is not industrially viable either.

Chlorination of 3,5-dichloro-2,4,6-trimethylphenol in trifluoroacetic acid is known to produce 3,4,5-trichloro-2,4,6-trimethyl-2,5-cyclohexadienone or 3,5,6-trichloro-2,4,6-trimethyl-2,4-cyclohexadienone. Antinori et al., J. Chem. Soc. (B), 373-77 (1969).

The invention is a process for preparing a 4-(bromo or chloro)methyl-3,5-dihalo-2,6-disubstituted phenol characterized by reacting a 4-methyl-2,6-di-substituted phenol with a brominating or chlorinating agent, in an aprotic organic diluent, under conditions whereby the 4-(bromo or chloro)methyl-3,5-dihalo-2,6-disubstituted phenol is prepared. The process is simple, highly selective and highly efficient. Characteristically, in general, the process requires no light nor the presence of base, and it can be run under moderate temperatures. The 4-(bromo or chloro)methyl-3,5-di- halo-2,6-disubstituted phenols are useful flame retardants, fungicides and monomer precursors for phenolic resins and engineering thermoplastics.

A side-chain halogenation process is known from the Journal of the Chemical Society, Perkin II, 1981, pages 32 to 41. In particaular, 2-methylphenol and 2,3,5,6-tetrabromo-4-methyl-phenol were halogenated in their methyl groups in one step by reaction with bromine under illumination. Surprisingly it is only the methyl group in 4-position which, in accordance with the invention, is halogenated whereas the substituents in the 4- and 6-positions remain unaffected. Moreover, in contrast to the reference process no light is required in the process of the invention.

For the purpose of the present invention, "halo" refers to any halogen, preferably fluorine, chlorine or bromine.

The 4-(bromo or chloro)methyl-3,5-dihalo-2,6-disubstituted phenols are a type of halogenated phenol. The 4-(bromo or chloro)methyl-3,5-dihalo-2,6-disubstituted phenols include compounds of the general formula

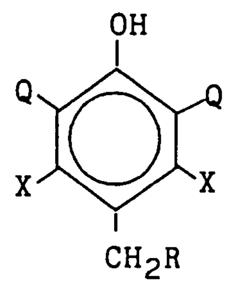

(I)

wherein

Q is separately at each occurrence an alkyl radical (with at most secondary carbon atom bonded directly to

the phenol ring) or an inertly-substituted alkyl, each preferably from 1 to 12 carbon atoms, more preferably Q is the alkyl and most preferably methyl;

X is separately at each occurrence a halogen atom, more preferably fluorine, bromine or chlorine, and most preferably bromine; and

R is bromine or chlorine and preferably bromine.

The most preferred of the 4-(bromo or chloro)methyl-3,5-dihalo-2,6-disubstituted phenols is 4-bromomethyl-3,5-dibromo-2,6-dimethylphenol.

The 4-methyl-2,6-disubstituted phenols include compounds of the general formula

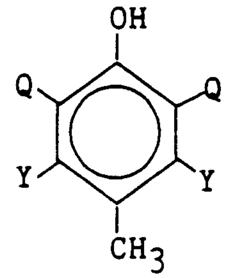

(II)

wherein

Q is as defined hereinbefore, and

Y is separately at each occurrence hydrogen or

X (as defined hereinbefore), preferably hydrogen.

The most preferred of the 4-methyl-2,6-disubstituted phenols is 2,4,6-trimethylphenol (mesitol).

The inertly-substituted alkyl (Q) moieties are those substituted alkyl moieties which do not, in general, interfere with the process for preparing the corresponding 4-(bromo or chloro)methyl-3,5-dihalo-2,6-disubstituted phenol. For example, the inertly-substituted alkyl moiety can be an alkyl moiety which is substituted with a moiety such as an ether moiety or a halo moiety.

The 4-methyl-2,6-disubstituted phenols can be commercially obtained or prepared by known procedures or by the process herein, which can be employed to prepare the 4-methyl-2,6-disubstituted phenols of the formula (II), for example, with the desired halo Y moiety. The 4-methyl-2,6-disubstituted phenols, for example, of the formula (II) with each Y hydrogen, are commercially available (for example, 2,4,6-trimethylphenol) or they can be prepared by known procedures, for example, by the processes disclosed by Norton et al., U.S. Patent 2,841,623 (1958); Walts et al., U.S. Patent 3,360,573 (1967); Van Sorge, U.S. Patents 3,751,488 (1973) and 3,764,630 (1973); Suzuki et al., U.S. Patents 3,899,540 (1975) and 3,976,702 (1976).

The brominating or chlorinating agent is a means for imparting a bromine or chlorine moiety to (at least the 4-methyl moiety of) the 4-methyl-2,6-disubstituted phenol to prepare the 4-(bromo or chloro)methyl-3,5-dihalo-2,6-disubstituted phenol. Thus, the brominating or chlorinating agent is a suitable source of bromine or chlorine moiety. The brominating and chlorinating agent can include bromine ($Br_2$) and chlorine ($Cl_2$), and compounds such as, for example, bromine chloride. The brominating and chlorinating agent can also be an organic halogenating agent such as, for example, N-bromosuccinimide (a suitable source of the bromo moiety) and sulfuryl chloride (a suitable source of the chloro moiety).

Preferably, the brominating or chlorinating agent is elemental bromine and elemental chlorine. Most preferably, it is elemental bromine.

The aprotic organic diluent is generally inert. Thus, the aprotic organic diluent does not, in general, interfere with the process to prepare the 4-(bromo or chloro)methyl-3,5-dihalo-2,6-disubstituted phenol. In general, the aprotic organic diluent is a liquid under the conditions of the process and preferably is a liquid at room temperature (25 ° C). The aprotic organic diluent is preferably considered generally such as a liquid solvent. The aprotic organic diluent includes diluents such as alkanes such as, cyclohexane; haloalkanes such as dichlorofluoromethane, methylene chloride, chloroform, carbon tetrachloride, bromochloromethane, ethylene dichloride; and halogenated aromatics such as ortho-dichlorobenzene.

Preferred of the aprotic organic diluents are the haloalkanes. Most preferably, the haloalkane is bromochloromethane.

In the practice of the invention, the 4-methyl-2,6-disubstituted phenol is contacted with the brominating or chlorinating agent. Conditions are those sufficient to prepare the 4-(bromo or chloro)-methyl-3,5-dihalo-2,6-disubstituted phenol.

Amounts of the brominating or chlorinating agent thus required can, in general, range from one atomic equivalent to ten atomic equivalents per mole of the 4-methyl-2,6-disubstituted phenol reactant. The atomic equivalents are based on the equivalents of bromo or chloro moiety generally available from the brominating or chlorinating agent for imparting to the 4-methyl-2,6-disubstituted phenol to prepare the 4-(bromo-or chloro)methyl-3,5-dihalo-2,6-disubstituted phenol. For example, one mole of elemental bromine generally contains one atomic equivalent of bromo moiety available for the imparting to the 4-methyl-2,6-disubstituted phenol. Typically, upon the bromination or chlorination, a by-product of hydrogen chloride or bromide is produced.

In general, the ratio of the atomic equivalents of the brominating or chlorinating agent employed per mole of the 4-methyl-2,6-disubstituted phenol (converted to any of the halogenated phenols) can be from 1:1 to 7:1, and with each Y moiety halo, for example, to 5:1. Preferably, with the Y moiety of the 4-methyl-2,6-disubstituted phenol each hydrogen, the ratio of the atomic equivalents of the halogenating agent employed per mole of the 4-methyl-2,6-disubstituted phenol (converted to any of the halogenated phenols) is from 3:1 to 5:1 and most preferably from 3.2:1 to 4:1. Thus, it is most preferred that at least one atomic equivalent of the brominating or chlorinating agent or slightly more is employed per molar equivalent of the 4-methyl moiety of the 4-methyl-2,6-disubstituted phenol.

A halogenating agent other than the brominating and chlorinating agent may be employed to impart other halo moieties, for example, to the 3 or 5 positions of the ring with hydrogen for a Y moiety. The other halogenating agent can thus be a fluorinating agent such as, for example, perchloryl fluoride ($ClO_3F$). The use of the brominating and chlorinating agent is preferred.

Amounts of the aprotic organic diluent thus required can, in general, vary over a wide range. Preferably, the amount is sufficient to prepare a slurry or solution with the reactants or products. Preferably, the ratio of moles of aprotic organic diluent employed per mole of the 4-methyl-2,6-disubstituted phenol is from 5:1 to 1000:1 and more preferably from 10:1 to 50:1. The aprotic organic diluent can be used to initially dilute either the 4-methyl-2,6-disubstituted phenol or halogenating agent, for example, the brominating or chlorinating agent, or a plurality of them.

Temperatures employed can vary over a broad range. Conveniently the reaction temperature is maintained between -120°C and 100°C, preferably between 0°C and 85°C, most preferably between 0°C and 35°C. In general, the higher the temperature is, the faster the rate of the reaction is.

Pressures employed can, in general, vary over a wide range. The pressure employed can be supra- or subatmospheric. However, ambient pressure is generally preferred during the preparation.

Time (duration) of the process can, in general, vary over a broad range, for example, from a few minutes to several days. Preferably, the time of carrying out the process is from about half an hour to ten hours and more preferably from one hour to seven hours. Most preferably, the time employed is from two to five hours, especially with the brominating or chlorinating agent such as elemental bromine because typically greater than ninety percent of the 4-methyl-2,6-disubstituted phenols are converted to the appropriate product within the span of this latter time period. Shorter times can typically be employed when employing more of the brominating or chlorinating agent.

The 4-(bromo or chloro)methyl-3,5-dihalo-2,6-disubstituted phenols can often be employed as thus prepared, especially such as with, for example, from 95 percent to 99 percent by weight purity, or can be further purified by known methods. Preferably, purification involves recrystallization such as, for example, from toluene, carbon tetrachloride, methylene chloride or bromochloromethane.

In general, the process is highly selective and efficient. Preferably, the conversion of the 4-methyl-2,6-disubstituted phenol to 4-methyl-3,5-dihalo-2,6-disubstituted phenol (converted product) is thus at least 50 percent by weight, and can be at least 65 percent, even at least 80 percent. Selectivity of the thus converted product to the 4-(bromo or chloro)-methyl-3,5-dihalo-2,6-disubstituted phenol itself can be as high as at least 80 percent by weight of the converted product, even as high as at least 90 percent of the converted product, more preferably at least 95 percent of the converted product. Selectivity of the isolated product as high as 98 percent by weight or above, including 99 percent, can be obtained even without recrystallization. Thug, the yield of the 4-(bromo or chloro)methyl-3,5-dihalo-2,6-disubstituted phenol can be high. Preferably, the yield is thus 40 percent by weight or greater, more preferably 50 percent or greater and most preferably 65 percent or greater. The yield of the 4-(bromo or chloro)methyl-3,5-dihalo-2,6-disubstituted phenol can thus even be as high as 75 percent, even 80 percent, or greater, by weight.

The following preferred sequence generally illustrates the overall process:

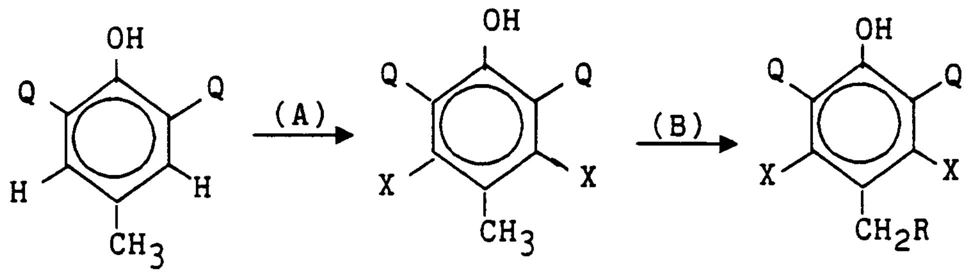

wherein Q, X and R are as defined hereinbefore;
the 4-methyl-2,6-disubstituted phenol of the step (A) is contacted with the halogenating agent, preferably the brominating or chlorinating agent, liberating corresponding hydrogen halide; and the 4-methyl-2,6-disubstituted phenol of the step (B) is contacted with the brominating or chlorinating agent, liberating corresponding hydrogen halide.
The 4-methyl-2,6-disubstituted phenol prepared in the step (A) can be isolated, if desired, purified and even reacted later in time. The step (B) alone is, of course, within the scope of the invention (with Y of the 4-methyl-2,6-disubstituted phenol of the formula (II) being X). Preferably,the process is carried out in the same reaction vessel by carrying out both of the steps (A) and (B) without special isolation of any halogenated intermediate 4-methyl-2,6-disubstituted phenol and generally without intermission of a significant time between the steps (A) and (B).

The following examples further illustrate the invention. Parts and percentages are by weight unless specified otherwise.

Example 1 - Preparation of 4-Bromomethyl--3,5-dibromo-2,6-dimethylpheol (Tribromomesitol)

136.2 g of 2,4,6-trimethylphenol (1.0 mole) (93 percent pure) was dissolved in 2.0 liters of carbon tetrachloride. Using a water bath for cooling, 205 ml of bromine (4.0 moles) was added at 20°C to 26°C over a period of 15 minutes. Hydrogen bromide gas was given off during the bromine addition, and a slurry of 3,5-dibromo-2,4,6-trimethylphenol was obtained.

The temperature was increased to 70°C to 75°C, and a solution was obtained. The solution was held at 70°C to 75°C for 3 hours. The unreacted bromine was removed by distillation with the aid of 1 liter of carbon tetrachloride. When 1 liter of solvent remained with the product, the solution was cooled to 25°C. The light brown solid which was obtained was filtered and dried under vacuum for 5 hours. A yield of 250 g (67 percent) was obtained, which analysis by gas chromatography showed to be 97 percent tribromomesitol and 3 percent dibromomesitol. The product has a melting point of 144°C to 146°C and has the following proton nuclear magnetic resonance spectrum ($CDCl_3$, δ: 2.30 (s, 6H), 4.90 (s, 2H), which is consistent with the structure of the product.

Example 2 - Process Variations: Amount of Brominating or Chlorinating Agent; Time

230-ml of bromine (4.5 moles) was added to 1.0 mole of 2,4,6-trimethylphenol (93 percent pure) in 2 liters of carbon tetrachloride; the refluxing period was 2 hours, and 260 g of product was obtained (70 percent conversion), otherwise following the work-up of Example 1. The crude solid was analyzed as 99 percent tribromomesitol.

Example 3 - Process Variations: Aprotic Organic Diluents; Temperatures

13.6 g of 2,4,6-trimethylphenol (0.1 mole) (99 percent pure) was dissolved in 200 ml of the aprotic organic diluent indicated hereinbelow, and 20.5 ml of bromine (0.4 moles) was added at 19°C to 23°C using a water bath for cooling. A solid formed which was slurried, and the slurry was heated at the reflux temperature as indicated hereinbelow for 2 hours. The excess bromine was then removed by distillation with the aid of 500 ml additional of the listed aprotic organic diluent, and the remaining diluent was removed under vacuum. The product was weighed and analyzed by gas chromatography for the conversion and selectivity of the product to 4-bromomethyl-3,5-dibromo-2,6-dimethylphenol, and the yield was calculated. The following was observed.

| Run | Diluent | °C Temp | grams of Conversion | % Selectivity | % Yield |
|---|---|---|---|---|---|
| A | cyclo-hexane | 76-78 | 34.0 | 63 | 63 |
| B | $CH_2Cl_2$ | 38-39 | 32.9 | 33 | 33 |
| C | $CHCl_3$ | 59-60 | 35.5 | 61 | 61 |
| D | $CCl_4$ | 71-74 | 37.2 | 83 | 83 |
| E | $(CH_2Cl)_2$ | 79-81 | 38.7 | 98 | 98 |
| F | $CH_2BrCl$ | 66-68 | 37.3 | 91 | 91 |

Example 4 - Pilot Scale Production

Two batches of high purity 4-bromomethyl-3,5-dibromo-2,6-dimethylphenol were efficiently prepared in a 100-gallon (376-liter) Pfaudler glass-lined reactor by the following procedures of Runs A and B. Of these, the procedure of Run B is preferred.

I. Preparation of the product

To start, 800 pounds (363 kg) of bromochloromethane was pumped into the reactor for each run. Next, 90 ° C (melted) mesitol was vacuum loaded into the reactor, 84.5 pounds (38.4 kg) in Run A and 113 pounds (51.3 kg) in Run B. Bromine, 347.5 pounds (157.7 kg) in Run A and 437 pounds (198 kg) in Run B, was steadily added over a period of time, 1 hour and 48 minutes in Run A and 2 hours and 8 minutes in Run B, with the reactor stirrer at 125-130 rotations per minute (rpm). Each procedure started out at 30 ° C and was somewhat above 60 ° C upon completion of the bromine addition. During each bromine addition, heat was applied to offset the cold (5 ° C to 10 ° C) reflux. Approximately 2/3 of the way through each bromine addition, the temperature was 50 ° C, and the batch briefly thickened and the air motor stirrer of the reactor dropped 6 rpm in Run A and 12 rpm in Run B. From this point on, the reaction slowed considerably.

The cook upon completion of each bromine addition lasted a period of time, 7 1/2 hours in Run A and 8 1/3 hours in Run B. Each cook started at 68 ° C, and the temperature upon completion of each cook was 73 ° C, and each batch was heated to keep the temperatures generally within this range, and maximum applied heating was carried out while keeping the reactor pressure less than 2 psig (a gauge pressure of less than 14 kPa) and condenser reflux less than 10 ° C by setting the water jacket temperature of the reflux condenser to 3 ° C.

Upon completion of each reaction, the reactor jacket was set at 60 ° C and propylene was sparged in, 9 pounds (4 kg) in Run A and 4 ½ pounds (2 kg) in Run B. Neutralization completion was indicated by loss of exotherm temperature and sudden disappearance of colored bromine vapors in the reactor headspace.

Upon completion of the neutralization, each product mixture was allowed to cool slowly, no faster than 20 ° C per hour. Product precipitation occurred at 28 ° C to 30 ° C in Run A and 35 ° C in Run B. Each batch was cooled to 2 ° C to 4 ° C and was dropped into a large batch filter and was next manually transferred to a 20-inch (50.8 cm) centrifuge, whereupon completion of centrifugation, solvent remained, 13 percent in Run A and 21.7 percent in Run B. A tan product resulted, 113 pounds (51.3 kg) in Run A (49 percent yield) and 182 pounds (82.6 kg) in Run B (59 percent yield).

B. Recrystallization

The centrifuged product of Runs A and B were combined, and in a clean 100-gallon Pfaudler reactor, 278 pounds (126 kg) of dry solids from uniformly mixing Runs A and B was slurried in 75 gallons (280 liters) of methylene chloride. The mixture was heated to 60 ° C. The pressure was 15 psig (a gauge pressure of 103 kPa). Dissolution occurred near 50 ° C, and the stirring was maintained at 100 rpm during the heating. The solution was cooled at 20 ° C per hour. Crystallization occurred at 44 ° C, whereupon the stirrer was

slowed to 60 rpm. Upon cooling to 20 ° C, refrigeration was applied to the reactor overnight. The slurry was centrifuged at -3 ° C and dried under vacuum in a 2-foot (61 cm) rotary cone Pfaudler dryer in 3 loads. The product was 212 pounds (96.2 kg) of a white, fluffy solid (76 percent recrystallization yield) which melts at 146 ° C to 147 ° C and was analyzed by gas chromatography to be more than 99 percent pure 4-bromomethyl-3,5-dibromo-2,6-dimethylphenol.

## Claims

1. A process to prepare a 4-(bromo or chloro)methyl-3,5-dihalo-2,6-disubstituted phenol (I) characterized by reacting a 4-methyl-2,6-disubstituted phenol of the formula (II)

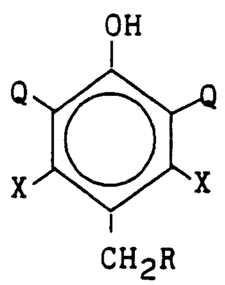

(I)

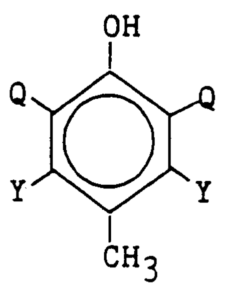

(II)

wherein
Q is separately at each occurrence in each formula, $C_{1-12}$ alkyl with the carbon bonded directly to the phenol ring selected from the group consisting of primary and secondary carbons, or
$C_{1-12}$ inertly-substituted alkyl;
Y is separately at each occurrence hydrogen or halogen atom.
X is separately at each occurrence a halogen atom and
R is separately at each occurrence bromine or chlorine.
with a brominating or chlorinating agent, in an aprotic organic diluent, under conditions whereby the 4-(bromo or chloro)methyl-3,5-dihalo-2,6-disubstituted phenol is prepared.

2. The process of Claim 1 wherein at least one Y is fluorine, chlorine or bromine.

3. The process of Claim 2 wherein the brominating or chlorinating agent is bromine or chlorine and the brominating or chlorinating agent is reacted with the 4-methyl-2,6-disubstituted phenol in a mole ratio of the brominating or chlorinating agent to 4-methyl-2,6-disubstituted phenol of from 1:1 to 7:1.

4. The process of Claim 3 wherein the aprotic organic diluent is a haloalkane.

5. The process of Claim 4 wherein the chlorinating or brominating agent is bromine and Y in formula II is bromine.

6. The process of Claim 4 wherein the aprotic organic diluent is bromochloromethane.

7. The process of any one of Claims 1 to 6 wherein the reaction is conducted at a temperature range of from -120 ° C to 100 ° C.

8. The process of any one of Claims 1 to 6 wherein the reaction is conducted at a temperature range of from 0 ° C to 85 ° C.

**Revendications**

1. Procédé de préparation d'un 4-(bromo ou chloro)-méthyl-3,5-dihalogéno-phénol-disubstitué en 2 et 6 (I), caractérisé par la réaction d'un 4-méthyl-phénol disubstitué en 2 et 6, de formule (II)

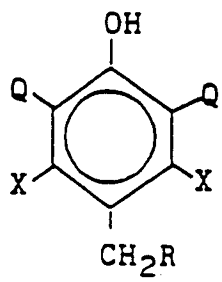

(I)

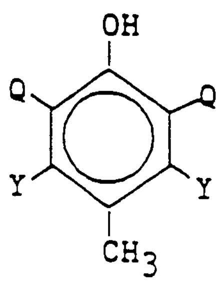

(II)

où Q est séparément, chaque fois qu'il se présente dans chaque formule, un groupe alkyle en $C_{1-12}$ dont l'atome de carbone directement lié au noyau phénolique est choisi dans le groupe comprenant les atomes de carbone primaire et secondaire,ou un groupe alkyle en $C_{1-12}$ substitué de manière inerte,
Y est séparément, chaque fois qu'il se présente, un atome d'hydrogène ou d'halogène,
X est séparément, chaque fois qu'il se présente, un atome d'halogène et
R est séparément, chaque fois qu'il se présente, un atome de brome ou de chlore,
avec un agent de bromation ou de chloration, dans un diluant organique aprotique, dans des conditions dans lesquelles on prépare le 4-(bromo ou chloro)méthyl-3,5-dihalogéno-phénol disubstitué en 2 et 6.

2. Procédé selon la revendication 1, dans lequel au moins un Y est un atome de fluor, de chlore ou de brome.

3. Procédé selon la revendication 2, dans lequel l'agent de bromation ou de chloration est le brome ou le chlore et dans lequel on fait réagir l'agent de bromation ou de chloration avec le 4-méthyl-phénol disubstitué en 2 et 6 dans un rapport molaire de l'agent de bromation ou de chloration au 4-méthyl-phénol disubstitué en 2 et 6 compris entre 1 : 1 et 7 : 1.

4. Procédé selon la revendication 3, dans lequel le diluant organique aprotique est un halogénoalcane.

5. Procédé selon la revendication 4, dans lequel l'agent de chloration ou de bromation est le brome et Y

dans la formule II est un atome de brome.

6. Procédé selon la revendication 4, dans lequel le diluant organique aprotique est le bromochlorométhane.

7. Procédé selon l'une quelconque des revendications 1 à 6, dans lequel on effectue la réaction à une température comprise entre - 120 ° C et 100 ° C.

8. Procédé selon l'une quelconque des revendications 1 à 6, dans lequel on effectue la réaction à une température comprise entre 0 ° C et 85 ° C.

**Patentansprüche**

1. Verfahren zur Herstellung von 4-(Brom oder Chlor)methyl-3,6-dihalogen-2,6-disubstituierten Phenolen (I) , dadurch gekennzeichnet, daß ein 4-Methyl-2,6-disubstituiertes Phenol der Formel (II)

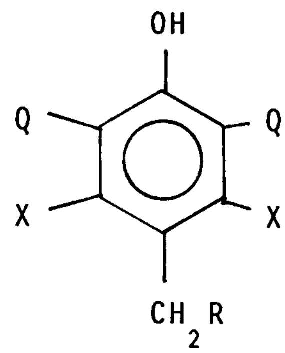

(I)

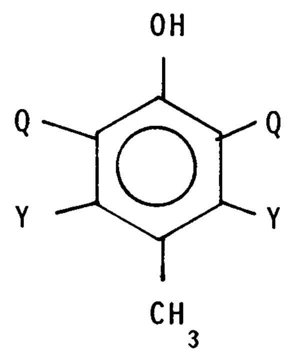

(II)

wobei die Substituenten Q unabhängig voneinander in jeder Formel jeweils einen, gegebenenfalls inert substituierten, $C_{1-12}$-Alkylrest, der mit einem primären oder sekundären Kohlenstoffatom an den Phenolring gebunden ist, bezeichnen,
die Substituenten Y unabhängig voneinander jeweils für ein Wasserstoff- oder Halogenatom stehen,
die Substituenten X unabhängig voneinander jeweils ein Halogenatom bezeichnen, und
R für ein Brom- oder Chloratom steht,
in einem aprotischen organischen Verdünnungsmittel mit einem Bromierungs- oder Chlorierungsmittel unter solchen Bedingungen umgesetzt wird, daß das 4-(Brom oder Chlor)-methyl-3,5-dihalogen-2,6-disubstituierte Phenol entsteht.

2. Verfahren nach Anspruch 1, dadurch gekennzeichnet, daß wenigstens einer der Substituenten Y Fluor, Chlor oder Brom ist.

3. Verfahren nach Anspruch 2, dadurch gekennzeichnet, daß das Bromierungs- oder Chlorierungsmittel Brom oder Chlor ist und das Bromierungs- oder Chlorierungsmittel mit dem 4-Methyl-2,6-disubstituierten Phenol in einem Molverhältnis von Bromierungs- oder Chlorierungsmittel zu 4-Methyl-2,6-disubstituiertem Phenol von 1:1 bis 7:1 umgesetzt wird.

**4.** Verfahren nach Anspruch 3, dadurch gekennzeichnet, daß das aprotische organische Verdünnungsmittel ein Halogenalkan ist.

**5.** Verfahren nach Anspruch 4, dadurch gekennzeichnet, daß das Bromierungs- oder Chlorierungsmittel Brom ist und Y in der Formel II Brom bezeichnet.

**6.** Verfahren nach Anspruch 4, dadurch gekennzeichnet, daß das aprotische organische Verdünnungsmittel Bromchlormethan ist.

**7.** Verfahren nach einem der Ansprüche 1 bis 6, dadurch gekennzeichnet, daß die Reaktion in dem Temperaturbereich zwischen -120 ° C und 100 ° C durchgeführt wird.

**8.** Verfahren nach einem der Ansprüche 1 bis 6, dadurch gekennzeichnet, daß die Reaktion in dem Temperaturbereich von 0 ° C bis 85 ° C durchgeführt wird.